(19) Europäisches Patentamt
European Patent Office
Office européen des brevets

(11) Publication number: **0 180 897 B1**

# (12) EUROPEAN PATENT SPECIFICATION

(45) Date of publication of patent specification: **07.08.91**

(51) Int. Cl.⁵: **C07H 19/073**, A61K 31/70

(21) Application number: **85113724.0**

(22) Date of filing: **29.10.85**

Divisional application 89102313.7 filed on 29/10/85.

The file contains technical information submitted after the application was filed and not included in this specification

## (54) 5-Fluorouracil derivatives.

(30) Priority: **30.10.84 JP 229938/84**
**30.11.84 JP 254587/84**
**17.01.85 JP 7190/85**
**25.03.85 JP 59788/85**
**09.05.85 JP 98295/85**
**30.08.85 JP 192582/85**
**03.09.85 JP 195223/85**

(43) Date of publication of application:
**14.05.86 Bulletin 86/20**

(45) Publication of the grant of the patent:
**07.08.91 Bulletin 91/32**

(84) Designated Contracting States:
**CH DE FR GB IT LI NL SE**

(56) References cited:
**EP-A- 0 009 882**
**EP-A- 0 129 984**
**FR-A- 2 428 052**
**GB-A- 2 066 812**
**GB-A- 2 072 164**

**CHEMICAL ABSTRACTS, vol. 101. 1984, page 657, ref. no. 55487y; Columbus, Ohio, US)**

(73) Proprietor: **OTSUKA PHARMACEUTICAL CO., LTD.**
**9, Kandatsukasacho 2-chome**
**Chiyoda-ku Tokyo 101(JP)**

(72) Inventor: **Fujii, Setsuro Aig de Blanche 703,**
**323, Ebiya-cho**
**Sanjyo-sagaru Gokomachi-dori**
**Nakagyo-ku Kyoto-shi Kyoto-fu(JP)**

(74) Representative: **Vossius & Partner**
**Siebertstrasse 4 P.O. Box 86 07 67**
**W-8000 München 86(DE)**

**EP 0 180 897 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to novel 5-fluorouracil derivatives, process for preparing the same and pharmaceutical compositions containing the same.

FR-A-2 428 052 discloses 5-fluoro-(β-uridine or 2'-deoxy-β-uridine) derivatives which can be used as carcinostatic agents. Chem. Abstr. 101:55487y describes 2'-deoxy-5-fluorouridine ether derivatives exhibiting anticarcinogenic activity. GB-A-2 066 812 discloses 5'-acyl uridine derivatives possessing strong antitumor activity with low toxicity. GB-A-2 072 164 describes 2'-deoxy-3',5'-di-O-alkylcarbonyl-5-fluorouridine derivatives which are useful as anti-tumor agents.

The 5-fluorouracil derivatives of the invention are novel compounds undisclosed in literature and having antitumor action.

The inventor has carried out research in an attempt to improve the antitumor action of known 5-fluorouracils, 2'-deoxy-5-fluorouridines and related compounds and to render them less toxic, and consequently succeeded in synthesizing a class of novel 5-fluorouracil derivatives and 5-fluorouridine derivatives which are substituted at 3'- or 5'- position with phenyl-lower alkyloxy group optionally having specific substituent(s) and related compounds. The inventor has also found that the compounds thus prepared have an excellent antitumor action and are very useful as antitumor agents.

The present invention provides a compound represented by the formula

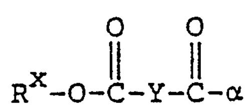

(1b)

wherein $R^x$ is a pyridyl group having 1 to 4 substituents selected from the group consisting of halogen atom, cyano group, hydroxy group and acyloxy group, Y is a phenylene group, and a is a group formed from a 5-fluorouracil derivative represented by the formula

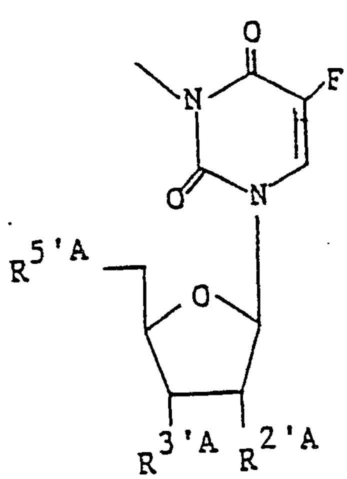

(1-1b)

wherein $R^{2'A}$ represents a hydrogen atom or a lower alkanoyloxy group, $R^{3'A}$ represents a lower alkanoyloxy group or a phenyl-lower alkyloxy group optionally substituted with 1 to 3 halogen atoms on the phenyl ring, and $R^{5'A}$ represents a hydrogen atom, a lower alkanoyloxy group or a phenyl-lower alkyloxy group optionally substituted with 1 to 3 halogen atoms on the phenyl ring, except for a compound wherein $R^{2'A}$ is a hydrogen, atom one of $R^{3'A}$ $R^{5'A}$ is a phenyl-lower alkyloxy group optionally substituted with 1 to 3 halogen atoms on the phenyl ring, and the other of $R^{3'A}$ and $R^{5'A}$ is a lower alkanoyloxy group.

Throughout the specification, the terms "lower alkyl" and "lower alkoxy" used as such or as contained in various functional groups are intended to exemplify $C_1$-$C_6$ straight or branched chain alkyl groups such as methyl, ethyl, propyl, isopropyl, butyl, tert-butyl, pentyl, hexyl, etc; and $C_1$-$C_6$ straight or branched chain alkoxy groups, such as methoxy, ethoxy, propoxy, isopropoxy, butoxy, tert-butoxy, pentyloxy, hexyloxy, etc., respectively. Further throughout the specification, the term "halogen" used as it is or as contained in various functional groups is intended to exemplify fluorine, chlorine, bromine, iodine, etc. Also throughout the specification, the term "lower alkylenedioxy" used as such or as contained in various functional groups

is meant to exemplify $C_1$-$C_4$ alkylenedioxy groups such as methylenedioxy, ethylenedioxy, trimethylenedioxy, tetramethylenedioxy, etc.

Examples of acyl oxy groups disclosed in the specification and claims and especially disclosed as the substituent of the pyridyl group represented by $R^X$ are various and include those having the following acyl moiety:

(i) $C_1$-$C_{20}$ alkanoyl groups optionally substituted with the substituents selected from the group consisting of halogen atom, hydroxy group, lower alkoxy group, aryloxy group, substituted or unsubstituted aryl group, phenyl-lower alkoxycarbonyl group and lower alkylcarbamoyl group.

(ii) arylcarbonyl groups optionally substituted with lower alkylenedioxy group or with 1 to 3 substituents selected from the group consisting of halogen atom, lower alkyl group, lower alkoxy group, carboxy group, lower alkoxycarbonyl group, nitro group, cyano group, phenyl-lower alkoxycarbonyl group, hydroxy group, guanidyl group, phenyl-lower alkoxy group, amino group and amino group substituted with lower alkyl group.

(iii) 5- or 6- membered unsaturated hetero ring-carbonyl groups having nitrogen atom, sulfur atom or oxygen atom as the hetero atom.

(iv) carbonic acid ester residue such as aryloxycarbonyl groups, straight or branched-chain or cyclic alkoxycarbonyl groups.

(v) substituted or unsubstituted cycloalkylcarbonyl groups.

(vi) lower alkenyl (or lower alkynyl)carbonyl groups.

(vii) lower alkenyl (or lower alkynyl)oxycarbonyl groups.

Examples of the acyl moieties included in the items (i) to (vii) are as follows.

(i) $C_1$-$C_{20}$ alkanoyl groups optionally substituted with the substituents selected from the group consisting of halogen atom, hydroxy group, lower alkoxy group, aryloxy group, substituted or unsubstituted aryl group, phenyl-lower alkoxycarbonyl group and lower alkylcarbamoyl group.

$C_1$-$C_{20}$ unsubstituted alkanoyl groups such as formyl, acetyl, propionyl, butyryl, isobutyryl, pentanoyl, hexanoyl, heptanoyl, octanoyl, nonanoyl, decanoyl, undecanoyl, dodecanoyl, tridecanoyl, tetradecanoyl, pentadecanoyl, hexadecanoyl, heptadecanoyl, ocatadecanoyl, nonadecanoyl, eicosanoyl, etc; $C_2$-$C_6$ alkanoyl groups substituted wth 1 to 3 halogen atoms such as monochloroacetyl, monobromoacetyl, dichloroacetyl, trichloroacetyl, 3-chloropropionyl, 4-chlorobutyryl, 5-chloropentanoyl, 6-chlorohexanoyl, etc; $C_2$-$C_6$ alkanoyl groups substituted with hydroxy group such as hydroxyacetyl, 3-hydroxypropionyl, 5-hydroxypentanoyl, 4-hydroxybutanoyl, 6-hydroxyhexanoyl, etc; $C_2$-$C_6$ alkanoyl groups substituted with $C_1$-$C_6$ alkoxy group such as methoxyacetyl, ethoxyacetyl, 3-propoxypropionyl, 6-hexyloxyhexanoyl, 3-methoxypropionyl, etc; $C_2$-$C_6$ alkanoyl groups substituted with phenoxy or naphthyloxy group such as phenoxyacetyl, 2-phenoxypropionyl, 3-phenoxypropionyl, 4-phenoxybutyryl, 5-phenoxypentanoyl, 6-phenoxyhexanoyl, α-naphthyloxyacetyl, etc; $C_2$-$C_6$ alkanoyl groups substituted with aryl group optionally substituted with 1 to 3 substituents selected from the group consisting of halogen atom, lower alkyl group, lower alkoxy group, carboxy group, lower alkoxycarbonyl group, nitro group and cyano group on the aryl ring (phenyl ring, naphthyl ring, etc.), such as α-phenylacetyl, 2-phenylpropionyl, 3-phenylpropionyl, 4-phenylbutyryl, 5-phenylpentanoyl, 6-phenylhexanoyl, α-(2-chlorophenyl)acetyl, α-(4-methylphenyl)acetyl, α-(3,4,5-trimethoxyphenyl)acetyl, α-(3,4-dimethoxyphenyl)acetyl, 6-(4-carboxyphenyl)hexanoyl, 4-(4-ethoxycarbonylphenyl)pentanoyl, α-(4-nitrophenyl)acetyl,α-(4-cyanophenyl)acetyl, α-naphthylacetyl, β-naphthylacetyl, etc; $C_1$-$C_{20}$ alkanoyl groups substituted with phenyl-$C_2$-$C_7$ lower alkoxycarbonyl group such as α-benzyloxycarbonylacetyl, 2-benzyloxycarbonylpropionyl, 3-benzyloxycarbonylpropionyl, 4-benzyloxycarbonylbutyryl, 5-benzyloxycarbonylpentanoyl, 6-benzyloxycarbonylhexanoyl, 3-(α-phenethyloxycarbonyl)propionyl, 3-(β-phenethyloxycarbonyl)propionyl, 5-(benzyloxycarbonyl)hexanoyl, 7-(benzyloxycarbonyl)heptanoyl, 8-(α-phenethyloxycarbonyl)octanoyl, 9-(β-phenethyloxycarbonyl)nonanoyl, 10-(benzyloxycarbonyl)decanoyl, 11-(β-phenethyloxycarbonyl)-tridecanoyl, 15-(benzyloxycarbonyl)pentadecanoyl, 17-(benzyloxycarbonyl)heptadecanoyl, 20-(benzyloxycarbonyl)eicosanoyl, etc; $C_1$-$C_{20}$ alkanoyl groups substituted with $C_2$-$C_7$ lower alkylcarbamoyl group such as methylcarbamoylacetyl, ethylcarbamoylacetyl, propylcarbamoylacetyl, butylcarbamoylacetyl, tert-butylcarbamoylacetyl, pentylcarbamoylacetyl, hexylcarbamoylacetyl, methylcarbamoylpropionyl, ethylcarbamoylbutyryl, propylcarbamoylpentanoyl, ethylcarbamoylhexanoyl, ethylcarbamoylheptanoyl, methylcarbamoyloctanoyl, ethylcarbamoylnonanoyl, methylcarbamoyldecanoyl, methylcarbamoytridecanoyl, ethylcarbamoylpentadecanoyl, methylcarbamoylheptadecanoyl, methylcarbamoyleicosaonyl, etc;

(ii) aryl-carbonyl groups optionally substituted with lower alkylenedioxy group or with 1 to 3 substituents selected from the group consisting of halogen atom, lower alkyl group, lower alkoxy group, carboxy group, lower alkoxycarbonyl group, nitro group, cyano group, phenyl-lower alkoxycarboxy group, hydroxy

group, guanidyl group, phenyl-lower alkoxy group, amino group and amino group substituted with lower alkyl group on the aryl ring.

aryl-carbonyl groups such as phenylcarbonyl, naphthylcarbonyl, etc. optionally substituted with lower alkylenedioxy group or with 1 to 3 substituents selected from the group consisting of halogen atom, lower alkyl group, lower alkoxy group, carboxy group, lower alkoxycarbonyl groups, nitro group, cyano group, phenyl-lower alkoxycarbonyl group, hydroxy group, guanidyl group, phenyl-lower alkoxy group, amino group and amino group substituted with lower alkyl group, such as benzoyl, α-naphthylcarbonyl, β-naphthylcarbonyl, 2-methylbenzoyl, 3-methylbenzoyl, 4-methylbenzoyl, 2,4-dimethylbenzoyl, 3,4,5-trimethylbenzoyl, 4-ethylbenzoyl, 2-methoxybenzoyl, 3-methoxybenzoyl, 4-methoxybenzoyl, 2,4-dimethoxybenzoyl, 3,4,5-trimethoxybenzoyl, 4-ethoxybenzoyl, 2-methoxy-4-ethoxybenzoyl, 2-propoxybenzoyl, 3-propoxybenzoyl, 4-propoxybenzoyl, 2,4-dipropoxybenzoyl, 3,4,5-tripropoxybenzoyl, 2-carboxybenzoyl, 3-carboxybenzoyl, 4-carboxybenzoyl, 2-chlorobenzoyl, 3-chlorobenzoyl, 4-chlorobenzoyl, 2,3-dichlorobenzoyl, 2,4,6-trichlorobenzoyl, 2-bromobenzoyl, 4-fluorobenzoyl, 2-methoxycarbonylbenzoyl, 3-methoxycarbonylbenzoyl, 4-methoxycarbonylbenzoyl, 2-ethoxycarbonylbenzoyl, 3-ethoxycarbonylbenzoyl, 4-ethoxycarbonylbenzoyl, 2-propoxycarbonylbenzoyl, 3-propoxycarbonylbenzoyl, 4-propoxycarbonylbenzoyl, 2-isopropoxycarbonylbenzoyl, 3-isopropoxycarbonylbenzoyl, 4-isopropoxycarbonylbenzoyl, 2-butoxycarbonylbenzoyl, 3-butoxycarbonylbenzoyl, 4-butoxycarbonylbenzoyl, 2-tert-butoxycarbonylbenzoyl, 3-tert-butoxycarbonylbenzoyl, 4-tert-butoxycarbonylbenzoyl, 2-pentyloxycarbonylbenzoyl, 3-pentyloxycarbonylbenzoyl, 4-pentyloxycarbonylbenzoyl, 2-hexyloxycarbonylbenzoyl, 3-hexyloxycarbonylbenzoyl, 4-hexyloxycarbonylbenzoyl, 3,5-dimethoxycarbonylbenzoyl, 3,4,5-trimethoxycarbonylbenzoyl, β-methyl-α-naphthylcarbonyl, α-methoxy-β-naphthylcarbonyl, β-chloro-α-naphthylcarbonyl, 2-cyanobenzoyl, 4-cyanobenzoyl, 2-nitrobenzoyl, 4-nitrobenzoyl, 2-benzyloxycarbonylbenzoyl, 3-benzyloxycarbonylbenzoyl, 4-benzyloxycarbonylbenzoyl, 3-(α-phenethyloxycarbonyl)benzoyl, 4-(β-phenethyloxycarbonyl)-benzoyl, 4-(3-phenylpropoxycarbonyl)benzoyl, 4-(6-phenylhexyloxycarbonyl)benzoyl, 2-hydroxybenzoyl, 3-hydroxybenzoyl, 2,3-dihydroxybenzoyl, 3,4-dihydroxybenzoyl, 3,4,5-trihydroxybenzoyl, 4-hydroxybenzoyl, 2-guanidylbenzoyl, 3-guanidylbenzoyl, 4-guanidylbenzoyl, 2-(benzyloxy)benzoyl, 3-(benzyloxy)-benzoyl, 4-(benzyloxy)benzoyl, 2-(α-phenethyloxy)benzoyl, 3-(α-phenethyloxy)benzoyl, 4-(α-phenethyloxy)benzoyl, 2-(β-phenethyloxy)benzoyl, 3-(β-phenethyloxy)benzoyl, 4-(β-phenethyloxy)-benzoyl, 4-(3-phenylpropoxy)benzoyl, 4-(4-phenylbutoxy)benzoyl, 4-(5-phenylpentyloxy)benzoyl, 4-(6-phenylhexyloxy)benzoyl, 2-aminobenzoyl, 3-aminobenzoyl, 4-aminobenzoyl, 2-methylaminobenzoyl, 3-methylaminobenzoyl, 4-methylaminobenzoyl, 2-ethylaminobenzoyl, 3-propylaminobenzoyl, 4-butylaminobenzoyl, 3-pentylaminobenzoyl, 4-hexylaminobenzoyl, 2-(N,N-dimethylamino)benzoyl, 3-(N,N-dimethylamino)benzoyl, 4-(N,N-dimethylamino)benzoyl, 3-(N-methyl-N-ethylamino)benzoyl, 4-(N,N-diethylamino)benzoyl, 3-(N,N-dipropylamino)benzoyl, 4-(N,N-dibutylamino)benzoyl, 4-(N,N-dipentylamino)benzoyl, 4-(N,N-dihexylamino)benzoyl, 3-benzyloxycarbonyl-1-naphthylcarbonyl, 6-(β-phenethyloxycarbonyl)-1-naphthylcarbonyl, 4-benzyloxycarbonyl-2-naphthylcarbonyl, 5-(α-phenethyloxycarbonyl)-2-naphthylcarbonyl, 2-hydroxy-1-naphthylcarbonyl, 3-hydroxy-1-naphthylcarbonyl, 4-hydroxy-1-naphthylcarbonyl, 5-hydroxy-1-naphthylcarbonyl, 6-hydroxy-1-naphthylcarbonyl, 7-hydroxy-1-naphtylcarbonyl, 8-hydroxy-1-naphthylcarbonyl, 1-hydroxy-2-naphthylcarbonyl, 4-hydroxy-2-naphthylcarbonyl, 5-hydroxy-2-naphthylcarbonyl, 7-hydroxy-2-naphthylcarbonyl, 2-guanidyl-1-naphtylcarbonyl, 3-guanidyl-1-naphthylcarbonyl, 5-guanidyl-1-naphthylcarbonyl, 6-guanidyl-1-naphthylcarbonyl, 8-guanidyl-1-naphthylcarbonyl, 1-guanidyl-2-naphthylcarbonyl, 4-guanidyl-2-naphthylcarbonyl, 6-guanidyl-2-naphthylcarbonyl, 8-guanidyl-2-naphthylcarbonyl, 2-amino-1-naphthylcarbonyl, 3-amino-1-naphthylcarbonyl, 4-amino-1-naphthylcarbonyl, 6-amino-1-naphthylcarbonyl, 4-amino-2-naphthylcarbonyl, 5-amino-1-naphthylcarbonyl, 7-amino-2-naphthylcarbonyl, 8-amino-2-naphthylcarbonyl, 3-(N,N-dimethylamino)-2-naphthylcarbonyl, 4-(N-methyl-N-ethylamino)-1-naphthylcarbonyl, 6-(N,N-dimethylamino)-1-naphthylcarbonyl, 7-(N-methyl-N-ethylamino)-2-naphthylcarbonyl, 8-(N-methyl-N-ethylamino)-1-naphthylcarbonyl, 3,4-methylenedioxybenzoyl, 3,4-ethylenedioxybenzoyl, 2,3-methylenedioxybenzoyl, etc.

(iii) 5- or 6- membered unsaturated hetero ring-carbonyl groups having nitrogen atom, sulfur atom or oxygen atom as the hetero atom.

thienylcarbonyl, furanylcarbonyl, thiazolylcarbonyl, quinolylcarbonyl, pyrazinylcarbonyl, pyridylcarbonyl, etc., such as 2-thienylcarbonyl, 3-thientylcarbonyl, 2-furanylcarbonyl, 3-furanylcarbonyl, 4-thiazolylcarbonyl, 2-quinolylcarbonyl, 2-pyrazinylcarbonyl, 2-pyridylcarbonyl, 3-pyridylcarbonyl, 4-pyridylcarbonyl, etc.

(iv) carbonic acid ester residue such as aryloxycarbonyl groups, straight or branched-chain or cyclic alkoxycarbonyl groups.

aryloxycarbonyl groups optionally substituted with 1 to 3 substituents selected from the group consisting of halogen atom, lower alkyl group and lower alkoxy group on the aryl ring (phenyl ring,

naphthyl ring, etc.), such as phenoxycarbonyl, α-naphthyloxycarbonyl, β-naphthyloxycarbonyl, 2-methylphenoxycarbonyl, 3-methylphenoxycarbonyl, 4-methylphenoxycarbonyl, 2,4-dimethylphenoxycarbonyl, 3,4,5-trimethylphenoxycarbonyl, 4-ethylphenoxycarbonyl, 2-methoxyphenoxycarbonyl, 3-methoxyphenoxycarbonyl, 4-methoxyphenoxycarbonyl, 2,4-dimethoxyphenoxycarbonyl, 3,4,5-trimethoxyphenoxycarbonyl, 4-ethoxyphenoxycarbonyl, 2-propoxyphenoxycarbonyl, 3-propoxyphenoxycarbonyl, 4-propoxyphenoxycarbonyl, 2,4-dipropoxyphenoxycarbonyl, 3,4,5-tripropoxyphenoxycarbonyl, 2-chlorophenoxycarbonyl, 3-chlorophenoxycarbonyl, 4-chlorophenoxycarbonyl, 2,3-dichlorophenoxycarbonyl, 2,4,6-trichlorophenoxycarbonyl, 2-bromophenoxycarbonyl, 4-fluorophenoxycarbonyl, β-methyl-α-naphthyloxycarbonyl, α-methoxy-β-naphthyloxycarbonyl, β-chloro-α-naphthyloxycarbonyl etc; straight or branched-chain or cyclic alkoxycarbonyl groups having 1 to 8 carbon atoms in the alkoxy moiety, such as methoxycarbonyl, ethoxycarbonyl, propoxycarbonyl, isopropoxycarbonyl, tert-butoxycarbonyl, butoxycarbonyl, pentyloxycarbonyl, hexyloxycarbonyl, cyolopropyloxycarbonyl, cyolobutyloxycarbonyl, cyolopentyloxycarbonyl, cyclohexyloxycarbonyl, cycloheptyloxycarbonyl, cyolooctyloxycarbonyl, etc.

(v) substituted or unsubstituted cycloalkyl carbonyl groups.

cycloalkylcarbonyl groups optionally substituted with halogen atom, hydroxy group, lower alkoxy group or lower alkyl group and having 3 to 8 carbon atoms in the cycloalkyl ring, such as cyclopropylcarbonyl, cyclobutylcarbonyl, cyclopentylcarbonyl, cyclohexylcarbonyl, cycloheptylcarbonyl, cyclooctylcarbonyl, 2-chlorocyclohexylcarbonyl, 3-hydroxycyclopentylcarbonyl, 3-methylcyclohexylcarbonyl, 4-methoxycyclohexylcarbonyl, etc.

(vi) lower alkenyl (or lower alkynyl)carbonyl groups.

carbonyl groups having $C_2$-$C_6$ alkenyl or alkynyl group, such as vinylcarbonyl, allylcarbonyl, 2-butenylcarbonyl, 3-butenylcarbonyl, 1-methylallylcarbonyl, 2-pentenylcarbonyl, 3-hexenylcarbonyl, ethynylcarbonyl, propynylcarbonyl, 2-butynylcarbonyl, 1-methyl-3-pentynylcarbonyl, 4-hexynylcarbonyl, etc.

(vii) lower alkenyl (or lower alkynyl)oxycarbonyl groups.

carbonyl groups having $C_2$-$c_6$ alkenyloxy or alkynyloxy group, such as vinyloxycarbonyl, allyloxycarbonyl, 2-butenyloxycarbonyl, 3-butenyloxycarbonyl, 2-hexenyloxycarbonyl, 1-methylallyloxycarbonyl, 2-pentenyloxycarbonyl, 3-hexenyloxycarbonyl, ethynyloxycarbonyl, propynyloxycarbonyl, 2-butynyloxycarbonyl, 1-methyl-3-pentynyloxycarbonyl, 4-hexynyloxycarbonyl, etc.

The pyridine ring represented by $R^x$ is substituted at any of the 2- to 6-positions with one to four of the substituents selected from the group consisting of hydroxy, halogen, cyano, and acyloxy.

Preferable examples of the group of the formula (Y) i.e.,

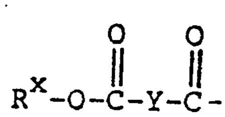

are those represented by the formula

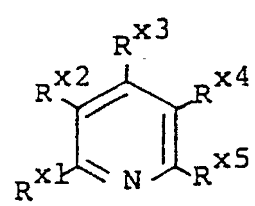

(Y-a)

wherein $R^{x1}$ is hydroxy or acyloxy; $R^{x2}$ and $R^{x4}$ are each hydrogen, halogen or cyano; $R^{x3}$ and $R^{x5}$ are each hydrogen, hydroxy or acyloxy; provided that at least one of $R^{x1}$, $R^{x3}$ and $R^{x5}$ represents a hydroxy group and that the hydrogen of one hydroxyl group represented by $R^{x1}$, $R^{x3}$ or $R^{x5}$ is substituted with a group

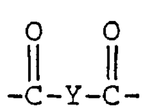

wherein Y is as defined above, and that at least one of $R^{x2}$, $R^{x3}$, $R^{x4}$ and $R^{x5}$ is (are) other than hydrogen.

The most preferable groups of the formula (Y) are those represented by the formula (Y-a) wherein $R^{x4}$ is halogen or cyano.

Examples of the pyridyl groups represented by $R^x$ and having the above various substituents are as follows.

2-hydroxy-3-pyridyl, 2-hydroxy-4-pyridyl,
2-hydroxy-5-pyridyl, 2-hydroxy-6-pyridyl,
2-hydroxy-5-chloro-3-pyridyl,
2-hydroxy-5-chloro-4-pyridyl,
4-hydroxy-5-chloro-2-pyridyl,
2-hydroxy-5-chloro-6-pyridyl,
2-hydroxy-5-fluoro-4-pyridyl,
4-hydroxy-5-fluoro-2-pyridyl,
2-hydroxy-5-fluoro-6-pyridyl,
2-hydroxy-5-bromo-4-pyridyl,
4-hydroxy-5-bromo-2-pyridyl,
2-hydroxy-5-bromo-6-pyridyl,
2-hydroxy-5-iodo-4-pyridyl,
4-hydroxy-5-iodo-2-pyridyl,
2-hydroxy-5-iodo-6-pyridyl,
2-hydroxy-3-bromo-4-pyridyl,
4-hydroxy-3-bromo-2-pyridyl,
2-hydroxy-3-chloro-4-pyridyl,
4-hydroxy-3-chloro-2-pyridyl,
2-hydroxy-3-chloro-6-pyridyl,
2-hydroxy-4-chloro-6-pyridyl,
2-acetyloxy-5-chloro-4-pyridyl,
2-acetyloxy-5-fluoro-4-pyridyl,
2-acetyloxy-5-bromo-4-pyridyl,
2-acetyloxy-5-iodo-4-pyridyl,
2-acetyloxy-5-cyano-4-pyridyl,
2-propanoyloxy-5-chloro-4-pyridyl,
2-butanoyloxy-5-chloro-4-pyridyl,
2-isobutanoyloxy-5-chloro-4-pyridyl,
2-pentanoyloxy-5-chloro-4-pyridyl,
2-hexanoyloxy-5-chloro-4-pyridyl,
3-acetyloxy-5-chloro-4-pyridyl,
2-acetyloxy-3-chloro-4-pyridyl,
4-acetyloxy-5-chloro-2-pyridyl,
4-acetyloxy-5-fluoro-2-pyridyl,
4-acetyloxy-5-bromo-2-pyridyl,
4-acetyloxy-5-iodo-2-pyridyl,
4-acetyloxy-5-cyano-2-pyridyl,
6-acetyloxy-5-chloro-2-pyridyl,
4-propanoyloxy-5-chloro-2-pyridyl,
4-butanoyloxy-5-chloro-2-pyridyl,
4-isobutanoyloxy-5-chloro-2-pyridyl,
4-pentanoyloxy-5-chloro-2-pyridyl,
4-hexanoyloxy-5-chloro-2-pyridyl,
4-acetyloxy-3-chloro-2-pyridyl,
3-acetyloxy-5-chloro-2-pyridyl,
2-acetyloxy-4-pyridyl,
2-propanoyloxy-4-pyridyl,
2-hexanoyloxy-4-pyridyl,
4-acetyloxy-2-pyridyl,
4-propanoyloxy-2-pyridyl,
4-hexanoyloxy-2-pyridyl,
2-methoxycarbonyloxy-5-chloro-4-pyridyl,
2-methoxycarbonyloxy-6-chloro-4-pyridyl,

2-ethoxycarbonyloxy-3-chloro-4-pyridyl,
2-ethoxycarbonyloxy-5-chloro-4-pyridyl,
2-ethoxycarbonyloxy-5-fluoro-4-pyridyl,
2-ethoxycarbonyloxy-5-bromo-4-pyridyl,
2-ethoxycarbonyloxy-6-chloro-4-pyridyl,
2-ethoxycarbonyloxy-5-chloro-3-pyridyl,
2-ethoxycarbonyloxy-5-chloro-6-pyridyl,
2-propoxycarbonyloxy-5-chloro-4-pyridyl,
2-hexyloxycarbonyloxy-5-chloro-4-pyridyl,
2-benzoyloxy-4-pyridyl,
3-benzoyloxy-4-pyridyl,
4-benzoyloxy-2-pyridyl,
3-benzoyloxy-2-pyridyl,
2-(2-methylbenzoyl)oxy-4-pyridyl,
2-(3-methylbenzoyl)oxy-4-pyridyl,
2-(4-methylbenzoyl)oxy-4-pyridyl,
2-(4-ethylbenzoyl)oxy-4-pyridyl,
2-(2-chlorobenzoyl)oxy-4-pyridyl,
2-(3-chlorobenzoyl)oxy-4-pyridyl,
2-(4-chlorobenzoyl)oxy-4-pyridyl,
2-(4-fluorobenzoyl)oxy-4-pyridyl,
2-(4-tert-butylbenzoyl)oxy-4-pyridyl,
2-(4-hexylbenzoyl)oxy-4-pyridyl,
4-(2-methylbenzoyl)oxy-2-pyridyl,
4-(4-ethylbenzoyl)oxy-2-pyridyl,
4-(3-chlorobenzoyl)oxy-2-pyridyl,
4-(4-fluorobenzoyl)oxy-2-pyridyl,
4-(4-tert-butylbenzoyl)oxy-2-pyridyl,
2-benzoyloxy-5-chloro-4-pyridyl,
2-benzoyloxy-5-fluoro-4-pyridyl,
2-benzoyloxy-5-bromo-4-pyridyl,
2-benzoyloxy-5-iodo-4-pyridyl,
2-benzoyloxy-5-cyano-4-pyridyl,
3-benzoyloxy-5-chloro-4-pyridyl,
2-benzoyloxy-3-chloro-4-pyridyl,
2-(2-methylbenzoyl)oxy-5-chloro-4-pyridyl,
2-(3-methylbenzoyl)oxy-5-chloro-4-pyridyl,
2-(4-methylbenzoyl)oxy-5-chloro-4-pyridyl,
2-(3,4,5-trimethylbenzoyl)oxy-5-chloro-4-pyridyl,
2-(4-ethylbenzoyl)oxy-5-chloro-4-pyridyl,
2-(4-tert-butylbenzoyl)oxy-5-fluoro-4-pyridyl,
2-(4-hexylbenzoyl)oxy-5-bromo-4-pyridyl,
2-(2-chlorobenzoyl)oxy-5-chloro-4-pyridyl,
2-(3-chlorobenzoyl)oxy-5-chloro-4-pyridyl,
2-(4-chlorobenzoyl)oxy-5-chloro-4-pyridyl,
2-(4-fluorobenzoyl)oxy-5-chloro-4-pyridyl,
2-(2,4-dichlorobenzoyl)oxy-5-chloro-4-pyridyl,
2-(3,4,5-trichlorobenzoyl)oxy-5-chloro-4-pyridyl,
2-(2-methoxybenzoyl)oxy-5-chloro-4-pyridyl,
2-(3-methoxybenzoyl)oxy-5-chloro-4-pyridyl,
2-(4-methoxybenzoyl)oxy-5-chloro-4-pyridyl,
2-(3,4,5-trimethoxybenzoyl)oxy-5-chloro-4-pyridyl,
4-benzoyloxy-5-chloro-2-pyridyl,
4-benzoyloxy-5-fluoro-2-pyridyl,
4-benzoyloxy-5-bromo-2-pyridyl,
4-benzoyloxy-5-iodo-2-pyridyl,
4-benzoyloxy-5-cyano-2-pyridyl,
3-benzoyloxy-5-chloro-2-pyridyl,

6-benzoyloxy-5-chloro-2-pyridyl,
6-(2,4-dichlorobenzoyloxy)-3-cyano-2-pyridyl,
6-(3,4,5-trimethoxybenzoyloxy)-3-cyano-2-pyridyl,
6-(4-fluorobenzoyloxy)-3-chloro-2-pyridyl,
6-benzoyloxy-3-cyano-2-pyridyl,
4-(4-methylbenzoyl)oxy-5-chloro-2-pyridyl,
4-(2,4-dimethylbenzoyl)oxy-5-chloro-2-pyridyl,
4-(3,4,5-trimethylbenzoyl)oxy-5-chloro-2-pyridyl,
4-(2-chlorobenzoyl)oxy-5-chloro-2-pyridyl,
4-(2,4-dichlorobenzoyl)oxy-5-chloro-2-pyridyl,
4-(4-methoxybenzoyl)oxy-5-chloro-2-pyridyl,
2-hydroxy-3-cyano-4-pyridyl,
2-hydroxy-3-cyano-5-pyridyl,
2-hydroxy-3-cyano-6-pyridyl,
2-hydroxy-5-cyano-6-pyridyl,
2-hydroxy-3,5-dichloro-4-pyridyl,
2-hydroxy-3,5-dichloro-6-pyridyl,
2-hydroxy-3,5-dibromo-4-pyridyl,
2-hydroxy-3,5-dibromo-6-pyridyl,
2,4-dihydroxy-6-pyridyl,
2,6-dihydroxy-4-pyridyl,
5-chloro-4-[p-(N,N-dimethylamino)benzoyloxy]-2-pyridyl,
5-bromo-4-[p-(N,N-dimethylamino)benzoyloxy]-2-pyridyl,
5-chloro-4-[p-(N,N-dimethylamino)benzoyloxy]-3-pyridyl,
5-chloro-4-[p-(N-methyl,N-ethylamino)benzoyloxy]-2-pyridyl,
5-fluoro-4-[o-(N,N-dimethylamino)benzoyloxy]-2-pyridyl,
5-fluoro-4-hexanoyloxy-2-pyridyl,
5-bromo-4-hexanoyloxy-2-pyridyl,
5-iodo-4-hexanoyloxy-2-pyridyl,
5-fluoro-4-octadecanoyloxy-2-pyridyl,
5-chloro-4-octadecanoyloxy-2-pyridyl,
5-bromo-4-octadecanoyloxy-2-pyridyl,
5-iodo-4-octadecanoyloxy-2-pyridyl,
5-fluoro-4-(2-furoyloxy)-2-pyridyl,
5-chloro-4-(2-furoyloxy)-2-pyridyl,
5-bromo-4-(2-furoyloxy)-2-pyridyl,
5-iodo-4-(2-furoyloxy)-2-pyridyl,
5-chloro-4-(3-furoyloxy)-2-pyridyl,
5-bromo-4-(3-furoyloxy)-2-pyridyl,
5-fluoro-4-(2-thenoyloxy)-2-pyridyl,
5-chloro-4-(2-thenoyloxy)-2-pyridyl,
5-bromo-4-(2-thenoyloxy)-2-pyridyl,
5-iodo-4-(2-thenoyloxy)-2-pyridyl,
5-chloro-4-(3-thenoyloxy)-2-pyridyl,
5-iodo-4-(3-thenoyloxy)-2-pyridyl,
4-(1-naphthoyloxy)-2-pyridyl,
4-(2-naphthoyloxy)-2-pyridyl,
5-(1-naphthoyloxy)-2-pyridyl,
5-(2-naphthoyloxy)-2-pyridyl,
6-(2-naphthoyloxy)-2-pyridyl,
3-cyano-6-(2-thenoyloxy)-2-pyridyl,
3-cyano-6-(3-thenoyloxy)-2-pyridyl,
4-cyano-6-(2-thenoyloxy)-2-pyridyl,
3-cyano-6-(2-furoyloxy)-2-pyridyl and the like.

Phenylene groups represented by Y in the pyridyloxycarbonyl-arylene carbonyl group of the formula (Y) include phenylene groups such as 1,2-phenylene, 1,3-phenylene, 1,4-phenylene and the like.

With respect to the formula (1b), the substituent $\alpha$ includes 5-fluorouracil derivative groups so far as it can be converted to 5-fluorouracil in vivo and so far as it can form an ester or amide linkage when taken

together with the carbonyl group to which this substituent α is attached; i.e, suitable 5-fluorouraoil derivative groups include those formed from the 5-fluorouracil derivatives represented by the compound of the formula (1-16).

The compound of the formula (1-1b) is disclosed in J. Med. Chem, 22, 1330, 1979, Japanese Unexamined Patent Publications No. 118800/1984 and No. 126221/1985.

With respect to the formula (1-1b),

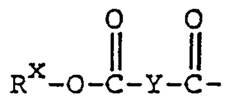

group can be linked by an amide linkage to the 3-position of the 5-fluorouracil ring.

With respect to the compounds of the formula (1-1b) the groups represented by $R^{2'A}$, $R^{3'A}$, $R^{5'A}$, are exemplified below.

Examples of lower alkanoyloxy groups are $C_1$-$C_6$ straight or branched chains alkanoyloxy groups such as formyloxy, acetyloxy, propionyloxy, butyryloxy, isobutyryloxy, pentanoyloxy, hexanoyloxy and the like.

Examples of phenyl-lower alkyl groups which constitute phenyl-lower alkoxy groups optionally having 1-3 halogens on the phenyl ring are phenylalkyl groups in which phenyl group optionally substituted with the above substituent is linked with $C_1$-$C_6$ alkylene group, such as 2-fluorobenzyl, 3-fluorobenzyl, 4-fluorobenzyl, 2,3-difluorobenzyl, 2,4-difluorobenzyl, 2,5-difluorobenzyl, 2-fluoro-3-chlorobenzyl, 2-fluoro-3-bromobenzyl, 2,6-difluorobenzyl, 2,3,4-trifluorobenzyl, 2,4,5-trifluorobenzyl, 2,3,5-trifluorobenzyl, 2,4,6-trifluorobenzyl, 3,4,5-trifluorobenzyl, 1-(2-fluorophenyl)ethyl, 2-(2-fluorophenyl)ethyl, 3-(3-fluorophenyl)propyl, 4-(2-fluorophenyl)butyl, 5-(2-fluorophenyl)pentyl, 6-(3-fluorophenyl)hexyl, 2-bromobenzyl, 3-bromobenzyl, 4-bromobenzyl, 2,3-dibromobenzyl, 2-bromo-3-fluorobenzyl, 2-fluoro-4-bromobenzyl, 2,4-dibromobenzyl, 2,5-dibromobenzyl, 2,6-dibromobenzyl, 2,3,4-tribromobenzyl, 2,4,5-tribromobenzyl, 2,3,5-tribromobenzyl, 2,4,6-tribromobenzyl, 3,4,5-tribromobenzyl, 1-(2-bromophenyl)ethyl, 2-(2-bromophenyl)ethyl, 3-(2-bromophenyl)-propyl, 4-(3-bromophenyl)butyl, 5-(2-bromophenyl)pentyl, 6-(4-bromophenyl)hexyl, 2-chlorobenzyl, 3-chlorobenzyl, 4-chlorobenzyl, 2,3-dichlorobenzyl, 2,4-dichlorobenzyl, 2,5-dichlorobenzyl, 2,6-dichlorobenzyl, 2-bromo-4-chlorobenzyl, 2-fluoro-4-chlorobenzyl, 2,3,4-trichlorobenzyl, 2,4,5-trichlorobenzyl, 2,3,5-trichlorobenzyl, 2,4,6-trichlorobenzyl, 3,4,5-trichlorobenzyl, 1-(4-chlorophenyl)ethyl, 2-(4-chlorophenyl)ethyl, 3-(2-chlorophenyl)propyl, 4-(4-chlorophenyl)butyl, 5-(3-chlorophenyl)pentyl, 6-(4-chlorophenyl)hexyl, 2-(3,4-dichlorophenyl)ethyl, 2-iodobenzyl, 3-iodobenzyl, 4-iodobenzyl, 3,4-diiodobenzyl, 3,4,5-triiodobenzyl, 2-(3-iodophenyl)ethyl, 6-(2-iodophenyl)hexyl and the like.

The compounds of the present invention can be prepared by the processes represented by the following reaction schemes (a) and (b).

Reaction scheme (a)

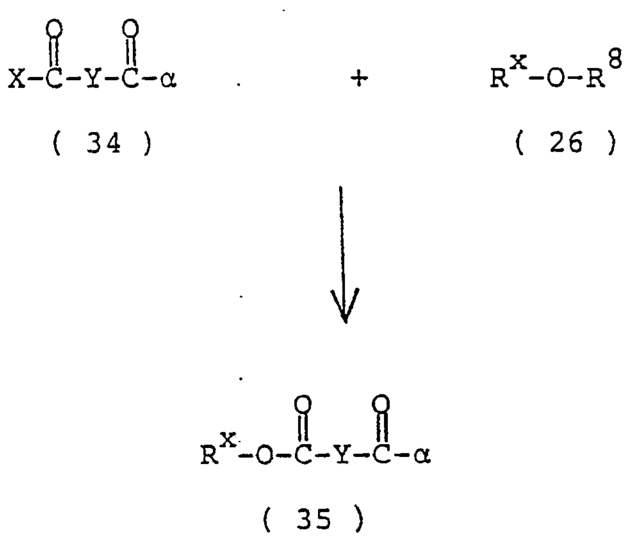

( 34 ) ( 26 )

( 35 )

wherein $R^8$ is hydrogen or tri(lower alkyl)silyl group, x is halogen, $\alpha$, $R^x$ and Y are as defined above.

This reaction can be carried out in the following manner.

First, acid halide (34) is prepared by introducing and acyl group XCO-Y-CO- into 3-position of the compound of the formula (1-1b) in accordance with the following reaction scheme (i).

Reaction scheme (i)

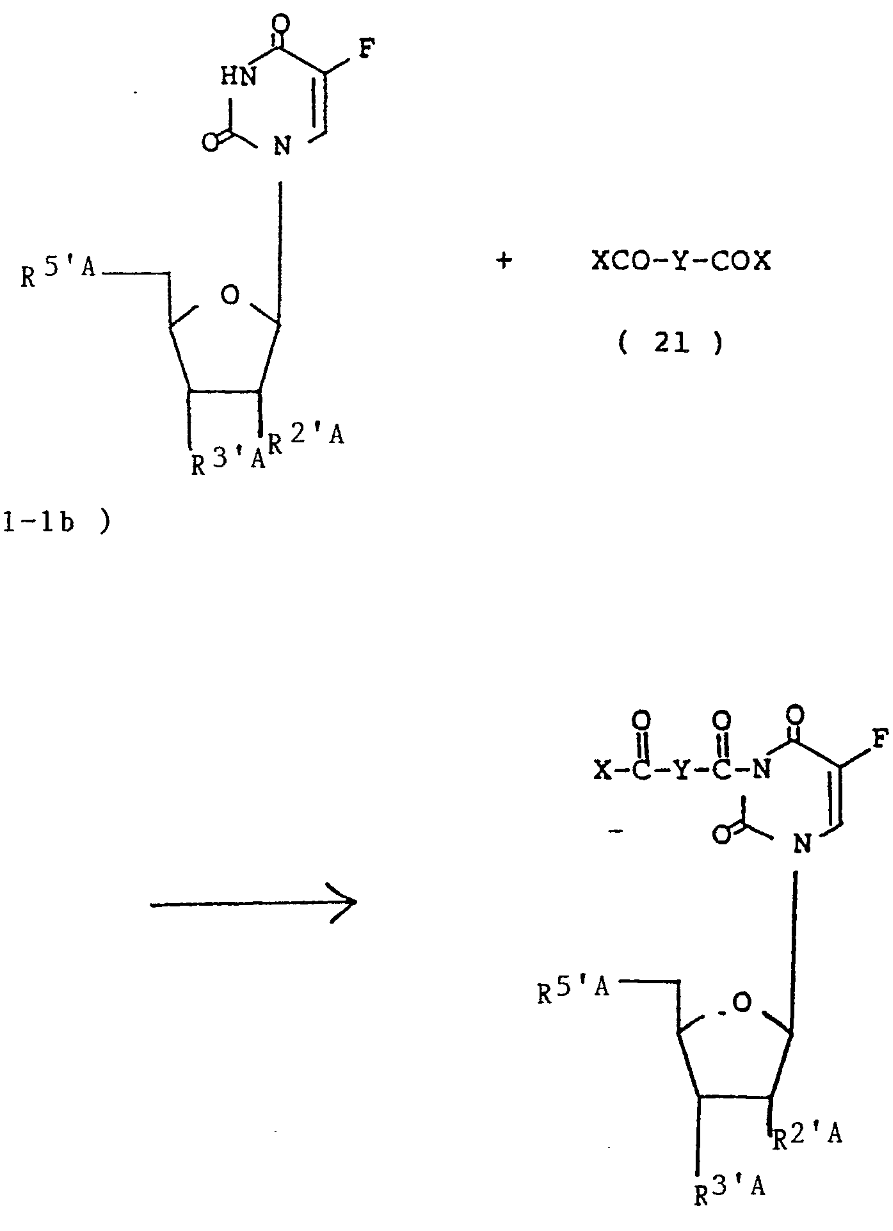

( 1-1b ) + XCO-Y-COX ( 21 )

( 34 )

wherein $R^{2'A}$, $R^{3'A}$, $R^{5'A}$ X and Y are as defined above.

The present reaction, wherein acyl (XCO-Y-CO- ) is introduced into the 3-position of the pyrimidine moiety (acylation), can be conducted by a usual process, e.g., the acid halide process. With the acid halide process, an acyl halide (XCO-Y-COX) is caused to act on the compound (1-1b) in a suitable solvent in the presence of an acid scavenger to give the compound (34). Examples of useful acid scavengers are sodium hydrogen carbonate, sodium carbonate, potassium carbonate, pyridine, triethylamine, etc. Examples of useful solvents are benzene, chloroform, methylene chloride, carbon tetrachloride, dioxane, tetrahydrofuran,

etc. The acyl halide is used in an amount of at least about one mole, preferably about 1 to about 3 moles, per mole of the compound (1-1b). The reaction temperature is usually -30 to 100° C, preferably room temperature to about 80° C. The reaction takes about 20 minutes to about 20 hours.

Then, the resulting compound (34) is reacted with the compound (26).

When the compound (26) used is one wherein $R^8$ is hydrogen, the reaction can be carried out in the same manner as in the preparation of compound (34).

Further when the compound (26) used is one wherein $R^8$ is tri(lower alkyl)silyl group, the reaction is conducted using a solvent, which will not adversely affect the reaction, in a dry state, preferably in an anhydrous state. Examples of preferred solvents are halogenated hydrocarbon such as dichloromethane and chloroform, aromatic hydrocarbons such as benzene and toluene, and aprotic solvents such as dioxane, acetonitrile, acetone and tetrahydrofuran. Usually, at least about one mole, preferably 1 to 3 moles, of the compound (26) is used per mole of the acid halide (34). The reaction is conducted at about 0 to about 100° C, preferably room temperature to about 80° C, for 1 to 6 hours, preferably about 2 hours. When $R^8$ is hydrogen, it is desirable to use a base, such as triethylamine, trimethylamine, dimethylaniline or pyridine, for the reaction. Further if $R^8$ is tri(lower alkyl)silyl, it is desirable to use a Lewis acid such as stannic chloride, zinc chloride or aluminum chloride.

Reaction scheme (b)

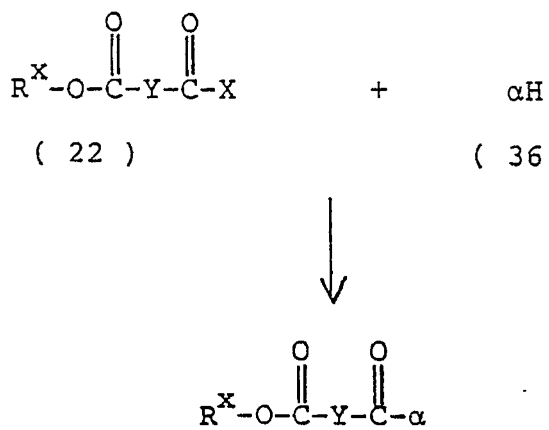

( 22 ) ( 36 )

( 35 )

wherein $R^x$, α, X and Y are as defined above.

This reaction can be carried out in the same manner as the reaction of the scheme (i).

The compounds (1b) of the present invention have an outstanding anticancer effect and are low in toxicity. For example, they are reduced in side effects such as loss of body weight. The present compounds are therefore very useful as antitumor agents for curing cancers in man and animals.

The desired products of the present invention are used in the form of generally acceptable pharmaceutical compositions which are prepared by using diluents and excipients such as fillers, bulking agents, binders, wetting agents, disintegrating agents, surfactants, lubricnats and the like. Administration unit forms of these pharmaceutical compositions of the present invention can be varied and selected so as to meet various therapeutical purposes. Typical forms of the pharmaceutical compositions can be exemplified such as tablets, pills, powders, liquids, suspensions, emulsions, granules, capsules, suppositories, injections (liquids, suspensions and others), ointments and the like.

In shaping into the form of tablets, those known as the carriers in this field can widely be applied for example, excipients such as lactose, purified sugar, sodium chloride, glucose, urea, starch, calcium carbonate, kaolin, crystalline cellulose, silicic acid and others; binders such as water, ethanol, propanol, simple syrup, a glucose solution, starch solution, gelatin solution, carboxymethyl cellulose, shellac, methyl cellulose, potassium phosphate, polyvinylpyrrolidone and others; disintegrating agents such as dried starch, sodium alginate, agar-agar powder, laminaran powder, sodium hydrogen carbonate, calcium carbonate, a fatty acid ester of polyoxyethylene sorbitan, sodium laurylsulfate, monoglyceride of stearic acid, starch, lactose and others; disintegration inhibitors such as purified sugar, stearing cacao butter, hydrogenated oils

and others; absorption accelerators such as quaternary ammonium base, sodium laurylsulfate and others; wetting agents such as glycerin, starch and others; adsorption accelerators such as starch, lactose, kaolin, bentonite, colloidal silicic acid and others; and lubricants such as purified talc powder, stearic acid salts, boric acid powder, polyethylene glycol and others can be exemplified. If necessary, the tablets can further be coated with usual coating film to make them into coated tablets, for example sugar-coated tablets, gelatin film-coated tablets, enteric film-coated tablets, film-coated tablets, or double-layered tablets, multiple-layered tablets and others. In shaping into the form of pills, those known as the carriers in this field can widely be applied for example, excipients such as glucose, lactose, starch, cacao butter, hydrogenated vegetable oils, kaolin, talc and others; binders such as powdered gum arabi, powdered tragacanth gum, gelatin, ethanol and others; disintegrating agent such as laminaran, agar-agar powder and others. In shaping into the form of suppositories, those known in this field can widely be applied for example, polyethylene glycol, cacao butter, a higher alcohol, an ester of a higher alcohol, gelatin, semi-synthesized glyceride and others. Capsules are prepared in a conventional manner by admixing the compound of the invention with the foregoing various carrier and encapsulating the mixture into hard-gelatin capsules, soft-gelatin capsules, etc. In case of preparing injections, solutions, emulsions and suspensions being prepared are sterilized, and they are preferably isotonic to the blood. In preparing into the form of solutions emulsions and suspensions, those known as the diluents in this field can widely be applied, for example water, ethanol, propylene glycol, ethoxylated isostearyl alcohol, polyoxylated isostearyl alcohol, a polyoxyethylene sorbitan fatty acid ester and others. In case of preparing isotonic solutions, sufficient amount of sodium chloride, glucose or glycerin may be added to make the solution to be isotonic to the blood. The pharmaceutical compositions for injection preparation may further be contain usual dissolving agents, buffer solutions, analgesic agents or the like if necessary. The pharmaceutical composition of the present invention may also contain coloring agents, preservatives, perfumes, seasoning agents, sweetening agents and others, as well as contain other medicines, if necessary. In shaping into the form of pastes, creams and gels, diluents such as white vaseline, paraffins, glycerine, cellulose derivatives, polyethylene glycols, silicones, bentonite and the like can be used.

The amount of the desired product according to the present invention to be contained as the active ingredient in the pharmaceutical composition is not specifically restricted and can be selected from a wide range, generally 1 to 70 % by weight, may be used.

Administration method of the above-mentioned pharmaceutical composition is not specifically restricted and can be administered through a suitable method for the respective types of administration forms, depending upon age of the patient, distinction of the sex and other conditions, conditions of the patient and others. For example, tablets, pills, liquids, suspensions, emulsions, granules and capsules are administered orally; injections are administered intraveneously singly or as a mixture with usual injectable transfusions such as a glucose solution, an amino acids solutions, and others; and if necessary the injections are administered singly intramuscularly, intracutaneously, subcutaneously or intraperitoneally; and the suppositories are administered into rectum.

The dosage of the desired products of the present invention may suitably be selected depending upon the method for administration, age of the patient, distinction of sex and other conditions, and conditions of the symptoms, and generally the pharmaceutical compositions of the invention can be administered in an amount of about 0.5 to about 20 mg/kg of the body weight/day, calculated as the compound of the invention (active ingredient), in 1 to 4 divided doses.

The present invention will be described in greater detail with reference to the following reference examples, examples, pharmaceutical tests and preparation examples.

In connection with the NMR data in the reference examples and examples, the numerals used as a subscript at the right of the symbol "C", "H" or "N" are used to refer to the position in the compound. Thus the term "$C_6$-H", for example, refers to the hydrogen bonded to the carbon atom at the 6-position. Similarly the term "$C_{3,4',5'}$-H", for example, denotes the hydrogens bonded to the carbon atoms at the 3'-, 4'- and 5'-positions. Also the term "$H_1$", for example, refers to the hydrogen bonded to the carbon atom at the 1-position. The term "$N_3$-H", for example, refers to the hydrogen bonded to the nitrogen atom at the 3-position of the 5-fluorouracil ring.

Reference Example 1

Preparation of 2-benzoyloxy-5-chloro-4-hydroxypyridine

A 10 ml quantity of hexamethyldisilazane was added to 1.00 g of 5-chloro-2,4-dihydroxy-pyridine, and the mixture was refluxed for 6 hours. Then excess hexamethyldisilazane was distilled off and the oily

residue was dissolved in 50 ml of dichloromethane. To the solution was added benzoyl chloride and 0.1 ml of stannic chloride and the mixture was stirred overnight at room temperature. The reaction mixture was neutralized with triethylamine, and the solvent was distilled off. Methanol was added to the residue and the mixture was stirred at room temperature for 2 hours. The solvent was distilled off and the residue was placed on a silica gel column and eluted with 2 % methanol-chloroform, giving the title compound.

The title compound softens at 184° C.

$^1$H-NMR (DMSO-$d_6$)δ:

8.27 (1N, s, $C_6$-H) 8.16-8.07 (2H, m,

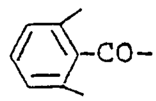 )

7.78-7.51 (3H, m,

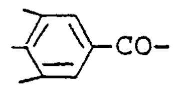 )

6.91 (1H, s, $C_3$-H)

Reference Example 2

Preparation of 2,4-bis(trimethylsilyloxy)-5-chloropyridine

A 50 ml quantity of hexamethyldisilazane was added to 9.6 g of 5-chloro-2,4-dihydroxy-pyridine, and the mixture was stirred overnight on an oil bath at 140° C. The insolubles were removed by filtration, and the filtrate was distilled under reduced pressure, giving 14.4 g of the title compound boiling at 120° C/7 mmHg. Yield 75 %.

Reference Example 3

Preparation of 2-acetoxy-5-chloro-4-hydroxy-pyridine

A 2.09 ml quantity of acetyl bromide and 0.10 ml of stannic chloride were added to a solution of 5.00 g of 2,4-bis(trimethylsilyloxy)-5-chloropyridine in 250 ml of dry dichloromethane, and the mixture was stirred at room temperature for 3.5 hours. The reaction mixture was neutralized with triethylamine, and the solvent was distilled off. The residue was applied to a silica gel column and eluted with 40 % ethyl acetate-benzene as an eluent, giving 2.07 g of the title compound in a yield of 64 %.

M.p. 270-272° C

$^1$H-NMR(DMSO-$d_6$)δ:

11.90 (1H, bs, OH)

8.20 (1H, s, $C_6$-H)

6.69 (1H, s, $C_3$-H)

2.27 (3H, s, $COCH_3$)

Reference Example 4

Preparation of 3-[3-(1,2-dihydro-2-oxo-4-pyridyloxycarbonyl)benzoyl]-5-fluoro-1-(2-tetrahydrofuranyl)uracil

A 1.00 g quantity of isophthaloyl chloride and 0.70 ml of triethylamine were added to a solution of 1.00 g of 5-fluoro-1-(2-tetrahydrofuranyl)uracil in 30 ml of dried dioxane. The mixture was refluxed for 2 hours. Thereto added was 1.00 ml of triethylamine and the mixture was refluxed for 2 hours. Thereto further added was 0.60 g of 4-hydroxy-2(1H)-pyridone and the mixture was refluxed for 3 hours. The solvent was distilled off and 30 ml of ethyl acetate and 30 ml of water were added to the residue. The insolubles were removed by filtration. The ethyl acetate layer was separated, dried over anhydrous sodium sulfate and concentrated.

The concentrate was subjected to silica gel column chromatography to conduct gradient elution using chloroform and mixtures of methanol (up to 2 %) and chloroform. The fractions corresponding to the title compound were collected and concentrated, giving 0.40 g of a powder in a yield of 18 %.
$^1$H-NMR(DMSO-$d_6$)δ:
11.74 (1H, bs, -NH-, disappeared by addition of $D_2O$) 8.63-8.41 (3H, m, $C_2$•$_4$•$_6$•-H of the benzoyl ring)
8.14 (1H, d, J = 7Hz, $C_6$-H)
7.84 (1H, t, J-= 8Hz, $C_3$-H of the benzoyl ring)
7.51 (1H, d, J = 8Hz, $C_6$-H of the pyridine ring)
6.34-6.26 (2H, m, $C_{3'5}$-H of the pyridine ring)
5.93 (1H, t, J = 4Hz, $C_{1'}$-H)
4.41-4.20 and 3.92-3.72 (each 1H, m, $C_{4'}$-H)
2.27-1.92 (4H, m, $C_{2'}$•$_{3'}$-H)

Example 1

Preparation of 3-[3-(4-benzoyloxy-5-chloro-2-pyridyloxycarbonyl)benzoyl]-2'-deoxy-3',5'-di-O-acetyl-5-fluorouridine

The title compound was prepared in the same manner as in Reference Example 4. $^1$H-NMR($CDCl_3$)δ:
8.68-8.17 (6H, m,

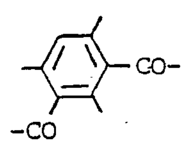 , 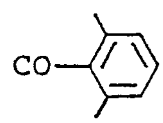

and $C_6$-H of the pyridine ring)
7.79-7.44 (5H, m,

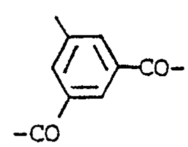 , 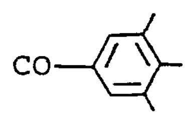

and $C_6$-H)
7.39 (1H, s, $C_3$-H of the pyridine ring)
6.27 (1H, t, J = 8Hz, $C_{1'}$-H)
5.29-5.17 (1H, m, $C_{3'}$-H)
4.51-4.28 (3H, m, $C_{4'}$•$_{5'}$-H)
2.70-2.08 (8H, m, $C_{2'}$-H and $COCH_3$ x 2)

Reference Example 5

Preparation of 3-[3-(2-acetoxy-5-chloro-4-pyridyloxycarbonyl)benzoyl]-5-fluoro-1-(2-tetrahydrofuranyl)uracil

A 5.53 ml quantity of triethylamine and 1.52 g of isophthaloyl chloride were added to a solution of 1.00 g of 1-(2-tetrahydrofuranyl)-5-fluorouracil in 50 ml of dried dioxane. The mixture was refluxed for 1 hour. The reaction mixture was filtered and the filtrate was concentrated. The concentrate was dissolved in 50 ml of acetonitrile. To the solution were added 3.46 ml of triethylamine and 1.40 g of 2-acetoxy-5-chloro-4-hydroxypyridine. The mixture was stirred at room temperature for 2 hours. The reaction mixture was filtered and concentrated. The concentrate was purified by silica gel column chromatography using chloroform as an eluent, giving 530 mg of the title compound in a yield of 20 %.
$^1$H-NMR($CDCl_3$)δ:
8.68-8.22 (3H, m,

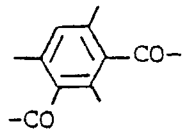 )

8.47 (1H, s, $C_6$-H of the pyridine ring) 7.72 (1H, t, J = 8Hz,

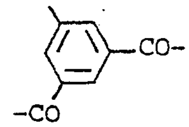 )

7.54 (1H, d, J = 6Hz, $C_6$-H)
7.27 (1H, s, $C_3$-H of the pyridine ring)
5.98-5.90 (1m, $C_{1'}$-H)
4.29-4.04 (2H, m, $C_{4'}$-H)
2.35 (3H, s, $COCH_3$)
2.43-1.89 (4H, m, $C_{2'}$•$_{3'}$-H)

Example 2

Preparation of 3-[3-(2-acetoxy-5-chloro-4-pyridyloxycarbonyl)benzoyl]-5'-deoxy-2',3'-di-O-acetyl-5-fluorouridine

The title compound was prepared in the same manner as in Reference Example 5. $^1$H-NMR($CDCl_3$)δ: 8.75-8.21 (4H, m,

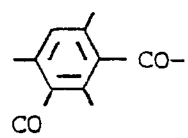

and $C_6$-H of the pyridine ring) 7.73 (1H, t, J = 8Hz,

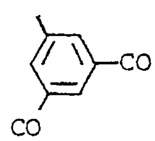 )

7.48 (1H, d, J = 6Hz, $C_6$-H)
7.26 (1H, s, $C_3$-H of the pyridine ring)
5.93 (1H, d, J = 5Hz, $C_{1'}$-H)
5.31 (1H, t, J = 6Hz, $C_{2'}$-H)
5.03 (1H, t, J = 6Hz, $C_{3'}$-H)
4.38-4.12 (1H, m, $C_{4'}$-H)
2.35 (3H, s, $COCH_3$ on the pyridine ring)
2.10 and 2.01 (each 3H, s, $COCH_3$)
1.46 (3H, d, J = 6Hz, $C_{5'}$-H)

Example 3

Preparation of 3-[3-(4-benzoyloxy-5-chloro-2-pyridyloxycarbonyl)benzoyl]-5-fluoro-2',3',5'-tri-O-acetyluridine

The title compound was prepared in the same manner as in Reference Example 5. $^1$H-NMR($CDCl_3$)δ:

8.75-8.18 (6H, m,

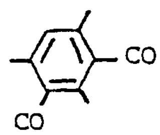 , 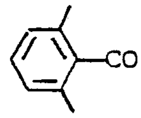

and $C_6$-H of the pyridine ring)
7.79-7.43 (5H, m, $C_6$-H)

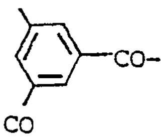 , 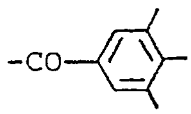 ,

7.39 (1H, s, $C_3$-H of the pyridine ring)
6.12-6.05 (1H, m, $C_{1'}$-H)
5.34-5.29 (2H, m, $C_{2'}{\bullet}_{3'}$-H)
4.38 (3H, bs, $C_{4'}{\bullet}_{5'}$-H)
2.18, 2.11, and 2.03 (each 3H, s, $COCH_3$)

Example 4

Preparation of 3-[3-(4-benzoyloxy-5-chloro-2-pyridyloxycarbonyl)benzoyl]-2'-deoxy-3',5'-di-O-benzyl-5-fluorouridine

The title compound was prepared in the same manner as in Reference Example 5. $^1$H-NMR($CDCl_3$)δ:
8.66-8.11 (7H, m,

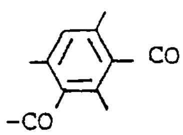 , 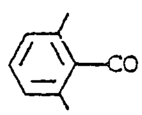 ,

$C_6$-H, $C_6$-H of the pyridine ring)
7.75-7.30 (15H, m,

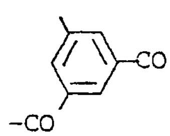 , 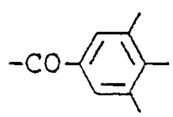 ,

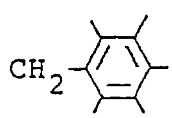

x 2 and $C_3$-H of the pyridine ring)
6.31 (1H, bt, J = 7Hz, $C_{1'}$-H)
4.56 (2H, s, $C_{5'}$

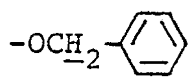

or $C_{3'}$

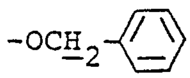 )

4.50 (2H, d, J = 2Hz, $C_{5'}$

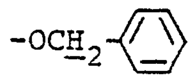

or $C_{3'}$

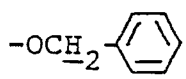 )

4.28-4.22 (2H, m, $C_{3'}$-H, $C_{4'}$-H)
3.91-3.52 (2H, m, $C_{5'}$-H)
2.66-2.04 (2H, m, $C_{2'}$-H)

Example 5

Preparation of 3-[3-(4-benzoyloxy-5-chloro-2-pyridyloxycarbonyl)benzoyl]-2'-deoxy-3',5'-di-O-(4-chlorobenzyl)-5-fluorouridine

The title compound was prepared in the same manner as in Reference Example 5.
$^1$H-NMR(CDCl$_3$)$\delta$:
8.64-8.17 (6H, m,

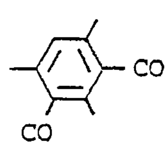 , 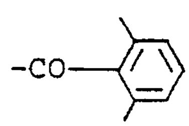

and $C_5$-H of the pyridine ring)
9.07 (1H, d, J = 6Hz, $C_6$-H)
7.77-7.13 (13H, m,

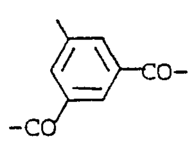 , 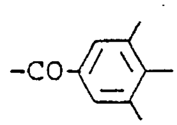 ,

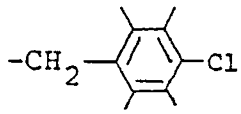

x 2 and $C_3$-H of the pyridine ring)
6.29 (1H, bt, J = 7Hz, $C_{1'}$-H)
4.52 (2H, s, $C_{5'}$

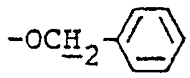

or $C_{3'}$

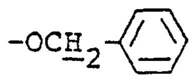

4.45 (2H, d, J = 2Hz, $C_{5'}$

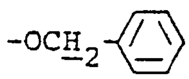

or $C_{3'}$

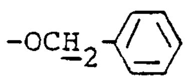 )

4.24-4.18 (2H, m, $C_{3'}$-H, $C_{4'}$-H)
3.90-3.51 (2H, m, $C_{5'}$-H)
2.67-2.03 (2H, m, $C_{2'}$-H)

Example 6

Preparation of 3-[3-(6-benzoyloxy-3-cyano-2-pyridyloxycarbonyl)benzoyl]-5'-deoxy-2',3'-di-O-acetyl-5-fluorouridine

The title compound was prepared in the same manner as in Reference Example 5.
$^1$H-NMR($CDCl_3$)δ:
8.77-8.14 (6H, m,

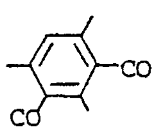 ,

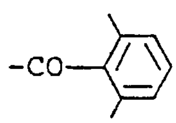

and $C_6$-H of the pyridine ring)
7.80-7.39 (6H, m,

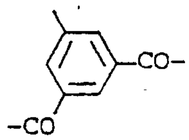 , 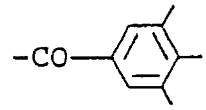 ,

$C_5$-H of the pyridine ring, $C_6$-H)
5.96 (1H, dd, J = 1Hz and J = 5Hz, $C_{1'}$-H)
5.32 (1H, t, J = 6Hz, $C_{2'}$-H)
5.03 (1H, t, J = 6Hz, $C_{3'}$-H)
4.30-4.18 (1H, m, $C_{4'}$-H)
2.09 (3H, s, $C_{2'}$ -$OCOCH_3$ or $C_{3'}$-$OCOCH_3$)
2.00 (3H, s, $C_{2'}$-$OCOC\underline{H_3}$ or $C_{3'}$-$OCOC\underline{H_3}$)
1.46 (3H, d, J = 6Hz, $\underline{C_{5'}}$-H)

Reference Example 6

Preparation of 3-[3-(1,2-dihydro-2-oxo-6-pyridyloxycarbonyl)benzoyl]-5-fluoro-1-(2-tetrahydrofuranyl)uracil

Isophthaloyl chloride (1.50 g) and 6.0 ml of triethylamine were added to a solution of 1.00 g of 5-fluoro-1-(2-tetrahydrofuranyl)uracil in 50 ml of dioxane. The mixture was refluxed for 1.5 hours. The resulting precipitate was removed by filtration and the filtrate was concentrated. To the concentrate was added 50 ml of methylene chloride and thereto added was 10 ml of a solution of 1.50 g of 2,6-bis(trimethylsilyloxy)-pyridine in methylene chloride. The mixture was left to stand at room temperature for 3 hours. The solvent was distilled off and the residue was subjected to silica gel column chromatography using as an eluent 2 % methanol-chloroform, giving 0.30 g of the title compound in a yield of 14 %. $^1$H-NMR($CDCl_3$)δ:
8.66-8.19 (3H, m,

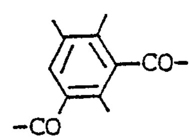 )

7.83-7.62 (2H, m,

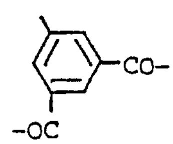

and $C_4$-H of the pyridine ring)
7.52 (1H, d, J = 6Hz, $C_6$-H)
6.77 and 6.68 (each 1H, d, J = 8Hz, $C_{3\bullet 5}$-H of the pyridine ring)
5.99-5.92 (1H, m, $C_{1'}$-H)
4.30-3.88 (2H, m, $C_{4'}$-H)
2.44-1.89 (4H, m, $C_{2'\bullet 3'}$-H)

Example 7

Preparation of 3-[3-(5-chloro-4-hydroxy-2-pyridyloxycarbonyl)benzoyl]-2'-deoxy-3',5'-di-O-acetyl-5-fluorouridine

The title compound was prepared in the same manner as in Reference Example 6. $^1$H-NMR($CDCl_3$)δ:
8.57-8.16 (4H, m,

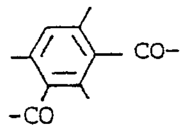

$C_6$-H of the pyridine ring)
7.80-7.53 (2H, m,

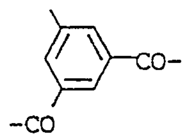

and $C_6$-H) 6.76 (1H, s, $C_3$-H of the pyridine ring)
6.19 (1H, bt, J = 7Hz, $C_{1'}$-H)
5.23-5.16 (1H, m, $C_{3'}$-H)
4.32-4.24 (3H, m, $C_{4'}$-H, $C_{5'}$-H)
2.60-1.85 (8H, m, $C_{2'}$-H, $COCH_3$ x 2)

Pharmacological Test I

(a) Sarcoma-180 subcultured in an ascites of ICR mice was diluted with a physiological saline solution and subcutaneously transplanted into the backs of ICR mice in an amount of 2 X $10^7$ each. Twenty-four hours after the transplantation, a test compound suspended in a 5 % solution of gum arabic was orally administered to each of mice once a day for 7 consecutive days.

The solid tumor was extirpated from under the dorsal skin of mice on the 10th day after the transplantation to measure the weight of the tumor. There was determined the ratio (T/C) of the weight of tumor (T) cut out from the group of mice treated with the test compound to the weight of tumor (C) from the group of mice not treated therewith. The 50 % tumor inhibition dose ($ED_{50}$ value) in which T/C is 0.5 was determined from the dose-response curve of dosage and the ratio (T/C). Table 1 shows the results.

Table 1 below lists the results together with the value obtained by using as a control an anti-cancer preparation containing 5-fluoro-1-(2-tetrahydrofuranyl)uracil and uracil in a ratio by weight of 1 to 4.

Table 1

| Test Compound | ED50 (mg/kg) |
|---|---|
| Compound of Example 1 | 7 |
| Compound of Example 2 | 12 |
| Compound of Example 3 | 22 |
| Compound of Example 4 | 5 |
| Control | 30 |

Preparation Example 1

| | |
|---|---|
| Compound of Example 1 | 0.025 mg |
| Starch | 132 mg |
| Magnesium stearate | 18 mg |
| Lactose | 50 mg |
| Total | About 200 mg |

Tablets were prepared in a conventional manner which each had the foregoing composition.

**Claims**

1. A compound represented by the formula

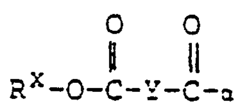

(1b)

wherein $R^x$ is a pyridyl group having 1 to 4 substituents selected from one group consisting of halogen atom, cyano group, hydroxy group and acyloxy group, Y is a phenylene group, and $\alpha$ is a group formed from a 5-fluorouracil derivative represented by the formula

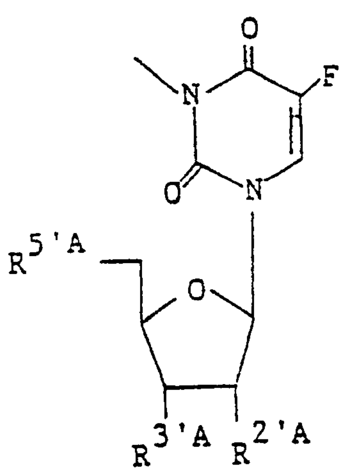

(1-1b)

wherein $R^{2'A}$ represents a hydrogen atom or a lower alkanoyloxy group, $R^{3'A}$ represents a lower alkanoyloxy group or a phenyl-lower alkyloxy group optionally substituted with 1 to 3 halogen atoms on the phenyl ring, and $R^{5'A}$ represents a hydrogen atom, a lower alkanoyloxy group or a phenyl-lower alkyloxy group optionally substituted with 1 to 3 halogen atoms on the phenyl ring, except for a compound wherein $R^{2'A}$ is a hydrogen atom, one or $R^{3'A}$ and $R^{5'A}$ is a phenyl-lower alkyloxy group optionally substituted with 1 to 3 halogen atoms on the phenyl ring, and the other of $R^{3'A}$ and $R^{5'A}$ is a lower alkanoyloxy group.

2. A compound according to claim 1 wherein the acyl residue of the acyloxy group as the substituent of the pyridyl group represented by $R^x$ is selected from the group consisting of:
(i) $C_1$-$C_{20}$ alkanoyl groups optionally substituted with the substituent selected from the group consisting of halogen atom, hydroxy group, lower alkoxy group, aryloxy group, substituted or unsubstituted aryl group, phenyl-lower alkoxycarbonyl group and lower alkylcarbamoyl group,

(ii) arylcarbonyl groups optionally substituted with a lower alkylenedioxy group or with 1 to 3 substituents selected from the group consisting of halogen atom, lower alkyl group, lower alkoxy group, carboxy group, lower alkoxycarbonyl group, nitro group, cyano group, phenyl-lower alkoxycarbonyl group, hydroxy group, guanidyl group, phenyl-lower alkoxy group, amino group and amino group substituted with lower alkyl group,
(iii) 5- or 6- membered unsaturated hetero ring-carbonyl groups having nitrogen atom, sulfur atom or oxygen atom as the hetero atom,
(iv) carbonic acid ester residue such as aryloxycarbonyl groups straight or branched-chain or cyclic alkoxycarbonyl groups,
(v) substituted or unsubstituted cycloalkylcarbonyl groups,
(vi) lower alkenyl (or lower alkynyl)carbonyl groups, and
(vii) lower alkenyl (or lower alkynyl)oxycarbonyl groups.

**3.** A compound according to claim 2 wherein the group

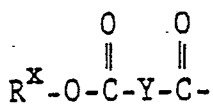

is a group represented by the formula

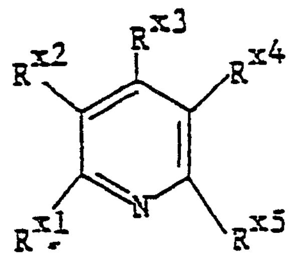

(Y-a)

wherein $R^{x1}$ is hydroxy or acyloxy; $R^{x2}$ and $R^{x4}$ are each hydrogen, halogen or cyano, $R^{x3}$ and $R^{x5}$ are each hydrogen, hydroxy or acyloxy, provided that at least one or $R^{x1}$, $R^{x3}$ and $R^{x5}$ represents $\alpha$ hydroxy group and that the hydrogen of one hydroxy group represented by $R^{x1}$, $R^{x3}$ or $R^{x5}$ is substituted with a group

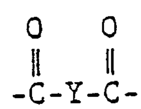

wherein Y is as defined above and that at least one of $R^{x2}$, $R^{x3}$, $R^{x4}$ and $R^{x5}$ is (are) other than hydrogen.

**4.** A compound according to claim 3 wherein $R^{x1}$ is hydroxy, benzoyloxy or $C_1$-$C_6$ alkanoyloxy, $R^{x2}$ is hydrogen atom, one of $R^{x3}$ and $R^{x5}$ is a hydrogen atom and the other of $R^{x3}$ and $R^{x5}$ is hydroxy, benzoyloxy or $C_1$-$C_6$ alkanoyloxy, and $R^{x4}$ is a cyano group or halogen atom, provided that at least one of $R^{x1}$, $R^{x3}$ and $R^{x5}$ is hydroxy and that the hydrogen of one hydroxy group represented by $R^{x1}$, $R^{x3}$ or $R^{x5}$ is substituted with a group

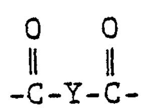

wherein Y is as defined above.

5. A compound according to claim 1 which is selected from the group consisting of 3-[3-(4-benzoyloxy-5-chloro-2-pyridyloxycarbonyl)benzoyl]-2'-deoxy-3',5'-di-O-acetyl-5-fluorouridine, 3-[3-(4-benzoyloxy-5-chloro-2-pyridyloxycarbonyl)benzoyl]-2'-deoxy-3',5'-di-O-benzyl-5-fluorouridine, 3-[3-(2-acetoxy-5-chloro-4-pyridyloxycarbonyl)benzoyl]-5'-deoxy-2',3'-di-O-acetyl-5-fluorouridine and 3-[3-(6-benzoyloxy-3-cyano-2-pyridyloxycarbonyl)benzoyl]-5'-deoxy-2',3'-di-O-acetyl-5-fluorouridine.

6. A process for preparing a compound represented by the formula

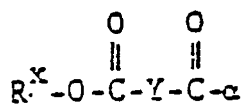

(Ib)

wherein $R^x$, Y and $\alpha$ are as defined in claim 1, characterized in that

a) the process comprises reacting a compound of the formula

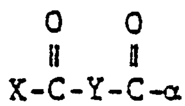

(34)

wherein X is a halogen atom and Y and $\alpha$ are as defined above, with a compound of the formula

$R^x$-O-$R^8$ (26)

wherein $R^x$ is as defined above, and $R^8$ is hydrogen or a tri(lower alkyl)silyl group, or

b) the process comprises reacting a compound of the formula

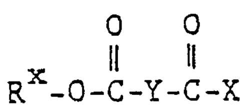

(22)

wherein $R^x$ and Y are as defined above and X is a halogen atom with a compound of the formula

$\alpha$H (36)

wherein $\alpha$ is as defined above.

7. A compound having the formula 1b according to claim 1 for use in the treatment of cancer.

8. A pharmaceutical composition comprising a compound having the formula 1b according to claim 1 and a pharmaceutically acceptable carrier.

## Revendications

1. Composé répondant à la formule

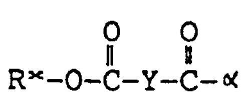

(1b)

dans laquelle $R^x$ représente un groupe pyridyle ayant 1 à 4 substituants choisis dans le groupe comprenant un atome d'halogène, un groupe cyano, un groupe hydroxy et un groupe acyloxy, Y représente un groupe phénylène et $\alpha$ représente un groupe formé d'un dérivé de 5-fluorouracile répondant à la formule

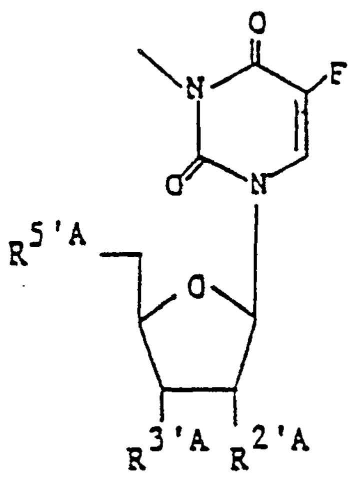

(1-1b)

dans laquelle $R^{2'A}$ représente un atome d'hydrogène ou un groupe alcanoyloxy inférieur, $R^{3'A}$ représente un groupe alcanoyloxy inférieur ou un groupe phényle-alkyloxy inférieur éventuellement substitué par 1 à 3 atomes d'halogène sur le cycle de phényle, et $R^{5'A}$ représente un atome d'hydrogène, un groupe alcanoyloxy inférieur ou un groupe phényle-alkyloxy inférieur éventuellement substitué par 1 à 3 atomes d'hydrogène sur le cycle de phényle, sauf pour un composé dans lequel $R^{2'A}$ représente un atome d'hydrogène, et un des radicaux $R^{3'A}$ et $R^{5'A}$ représente un groupe phényle-alkyloxy inférieur éventuellement substitué par 1 à 3 atomes d'halogène sur le cycle de phényle et l'autre radical $R^{3'A}$ ou $R^{5'A}$ représente un groupe alcanoyloxy inférieur.

**2.** Composé selon la revendication 1, caractérisé en ce que le résidu acyle du groupe acyloxy comme substituant du groupe pyridyle représenté par $R^x$ est choisi dans le groupe comprenant :

(i) des groupes alcanoyle en $C_1$-$C_{20}$ éventuellement substitués par le substituant choisi dans le groupe comprenant un atome d'halogène, un groupe hydroxy, un groupe alcoxy inférieur, un groupe aryloxy, un groupe aryle substitué ou non substitué, un groupe phényle-alcoxycarbonyle inférieur et un groupe alkylcarbamoyle inférieur,

(ii) des groupes arylcarbonyle éventuellement substitués par un groupe alkylènedioxy inférieur ou par 1 à 3 substituants choisis dans le groupe comprenant un atome d'halogène, un groupe alkyle inférieur, un groupe alcoxy inférieur, un groupe carboxy, un groupe alcoxycarbonyle inférieur, un groupe nitro, un groupe cyano, un groupe phényle-alcoxycarbonyle inférieur, un groupe hydroxy, un groupe guanidyle, un groupe phényle-alcoxy inférieur, un groupe amino et un groupe amino substitué par un groupe alkyle inférieur,

(iii) des groupes carbonyle hétérocycliques non saturés à 5 ou 6 chaînons, ayant un atome d'azote, un atome de soufre ou un atome d'oxygène comme hétéroatome,

(iv) un résidu d'ester de l'acide carbonique, comme des groupes aryloxycarbonyle, des groupes alcoxycarbonyle à chaîne droite ou ramifiée, ou cycliques,

(v) des groupes cycloalkylcarbonyle substitués ou non substitués,

(vi) des groupes alkényle inférieur-carbonyle ou alkynyle inférieur-carbonyle, et

(vii) des groupes alkényle inférieur-oxycarbonyle ou alkynyle inférieur-oxycarbonyle.

**3.** Composé selon la revendication 2, caractérisé en ce que le groupe

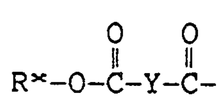

est un groupe répondant à la formule

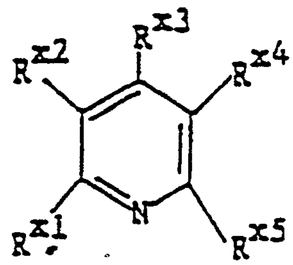

(Y-a)

dans laquelle $R^{x1}$ représente un hydroxy ou un acyloxy ; $R^{x2}$ et $R^{x4}$ représentent chacun un atome d'hydrogène, un atome d'halogène ou un groupe cyano ; $R^{x3}$ et $R^{x5}$ représentent chacun un atome d'hydrogène, un groupe hydroxy ou acyloxy, à condition qu'au moins un des radicaux $R^{x1}$, $R^{x3}$ et $R^{x5}$ représente un groupe hydroxy et que l'atome d'hydrogène d'un groupe hydroxy représenté par $R^{x1}$, $R^{x3}$ ou $R^{x5}$ soit substitué par un groupe

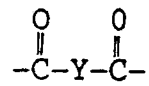

dans lequel Y a la signification indiquée ci-dessus et qu'au moins un des radicaux $R^{x2}$, $R^{x3}$, $R^{x4}$ et $R^{x5}$ représente autre chose qu'un atome d'hydrogène.

**4.** Composé selon la revendication 3, caractérisé en ce que $R^{x1}$ représente un hydroxy, un benzoyloxy ou un alcanoyloxy en $C_1$-$C_6$, $R^{x2}$ représente un atome d'hydrogène, un des radicaux $R^{x3}$ et $R^{x5}$ représente un atome d'hydrogène et l'autre radical $R^{x3}$ ou $R^{x5}$ représente un hydroxy, un benzoyloxy ou un alcanoyloxy en $C_1$-$C_6$, et $R^{x4}$ représente un groupe cyano ou un atome d'halogène, à condition qu'au moins un des radicaux $R^{x1}$, $R^{x3}$ et $R^{x5}$ représente un hydroxy et que l'atome d'hydrogène d'un groupe hydroxy représenté par $R^{x1}$, $R^{x3}$ ou $R^{x5}$ soit substitué par un groupe

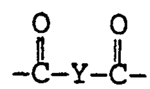

dans lequel Y a la signification indiquée ci-dessus.

**5.** Composé selon la revendication 1, caractérisé en ce qu'on le choisit dans le groupe comprenant la 3-[3-(4-benzoyloxy-5-chloro-2-pyridyloxycarbonyl)benzoyl]-2'-désoxy-3',5'-di-O-acétyl-5-fluorouridine, la 3-[3-(4-benzoyloxy-5-chloro-2-pyridyloxycarbonyl)benzoyl]-2'-désoxy-3',5'-di-O-benzyl-5-fluorouridine, la 3-[3-(2-acétoxy-5-chloro-4-pyridyloxycarbonyl)benzoyl]-5'-désoxy-2',3'-di-O-acétyl-5-fluorouridine et la 3-[3-(6-benzoyloxy-3-cyano-2-pyridyloxycar bonyl)benzoyl]-5'-désoxy-2',3'-di-O-acétyl-5-fluorouridine.

**6.** Procédé de préparation d'un composé répondant à la formule

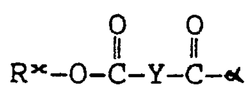

(1b)

dans laquelle $R^x$, Y et $\alpha$ ont la même signification que dans la revendication 1, caractérisé en ce que

a) le procédé comprend la réaction d'un composé de formule

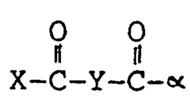

(34)

dans laquelle X représente un atome d'halogène et Y et α ont la même signification que précédemment, avec un composé de formule

$R^x$-O-$R^8$ (26)

dans laquelle $R^x$ a la même signification que précédemment et $R^8$ représente un atome d'hydrogène ou un groupe tri(alkyle inférieur)silyle, ou
b) le procédé comprend la réaction d'un composé de formule

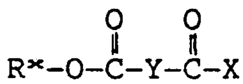

(22)

dans laquelle $R^x$ et Y ont la même signification que précédemment et X représente un atome d'halogène, avec un composé de formule

αH (36)

dans laquelle α a la même signification que précédemment.

7. Composé répondant à la formule 1b selon la revendication 1, pour une utilisation dans le traitement du cancer.

8. Composition pharmaceutique comprenant un composé répondant à la formule 1b selon la revendication 1 et un support pharmaceutiquement acceptable.

**Patentansprüche**

1. Verbindung der Formel

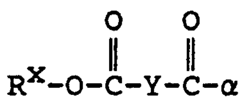

(1b)

in der $R^x$ eine Pyridylgruppe mit 1 bis 4 Substituenten ausgewählt aus Halogenatomen, der Cyano- oder Hydroxygruppe oder Acyloxyresten bedeutet, Y eine Phenylengruppe darstellt und α eine aus einem 5-Fluoruracilderivat erhaltene Gruppe ist, dargestellt durch die Formel

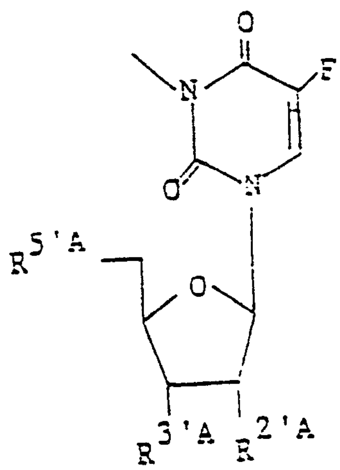

(1-1b)

bedeutet, in der $R^{2'A}$ ein Wasserstoffatom oder einen Niederalkanoyloxyrest darstellt, $R^{3'A}$ einen

Niederalkanoyloxy- oder einen gegebenenfalls mit 1 bis 3 Halogenatomen am Phenylring substituierten Phenylniederalkyloxyrest darstellt und $R^{5'A}$ ein Wasserstoffatom, einen Niederalkanoyloxyrest oder einen gegebenenfalls mit 1 bis 3 Halogenatomen am Phenylring substituierten Phenylniederalkyloxyrest bedeutet, ausgenommen eine Verbindung, in der $R^{2'A}$ ein Wasserstoffatom bedeutet, einer der Reste $R^{3'A}$ und $R^{5'A}$ ein gegebenenfalls mit 1 bis 3 Halogenatomen am Phenylring substituierter Phenylniederalkyloxyrest ist und der andere der Reste $R^{3'A}$ und $R^{5'A}$ ein Niederalkanoyloxyrest ist.

2. Verbindung nach Anspruch 1, wobei der Acylrest der Acyloxygruppe als Substituent der durch $R^x$ wiedergegebenen Pyridylgruppe ausgewählt ist aus:
(i) gegebenenfalls mit einem Halogenatom, einer Hydroxygruppe, einem Niederalkoxy- oder Aryloxyrest, einem substituierten oder nicht-substituierten Arylrest, einem Phenylniederalkoxycarbonylrest oder einem Niederalkylcarbamoylrest substituierten $C_1$-$C_{20}$-Alkanoylresten,
(ii) Arylcarbonylresten, gegebenenfalls mit einem Niederalkylendioxyrest oder mit 1 bis 3 Substituenten, ausgewählt aus Halogenatomen, Niederalkyl- oder Niederalkoxyresten, der Carboxylgruppe, Niederalkoxycarbonylresten, der Nitro- oder Cyanogruppe, Phenylniederalkoxycarbonylresten, der Hydroxy- oder Guanidylgruppe, Phenylniederalkoxyresten, der Aminogruppe oder mit Niederalkylresten substituierten Aminogruppen substituiert,
(iii) 5 bis 6 gliedrigen ungesättigten Heteroringcarbonylgruppen mit Stickstoff, Schwefel oder Sauerstoff als Heteroatom,
(iv) Kohlensäureesterresten, wie Aryloxycarbonylreste, gerad- oder verzweigtkettige oder cyclische Alkoxycarbonylreste,
(v) substituierten oder unsubstituierten Cycloalkylcarbonylresten,
(vi) Niederalkenyl- (oder Niederalkinyl)carbonylresten, und
(vii) Niederalkenyl- (oder Niederalkinyl)oxycarbonylresten.

3. Verbindung nach Anspruch 2, in der die Gruppe

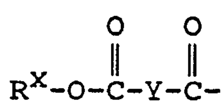

eine Gruppe bedeutet, die durch die Formel

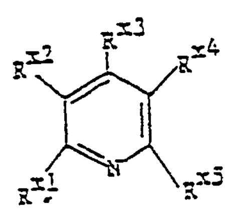

(Y-a)

wiedergegeben wird, in der $R^{x1}$ eine Hydroxygruppe oder einen Acyloxyrest bedeutet, $R^{x2}$ und $R^{x4}$ jeweils Wasserstoff- oder Halogenatome oder Cyanogruppen darstellen, $R^{x3}$ und $R^{x5}$ jeweils Wasserstoffatome, Hydroxygruppen oder Acyloxyreste bedeuten, mit der Maßgabe, daß mindestens einer der Reste $R^{x1}$, $R^{x3}$ und $R^{x5}$ eine Hydroxygruppe darstellt und daß das Wasserstoffatom einer der durch die Reste $R^{x1}$, $R^{x3}$ oder $R^{x5}$ wiedergegebenen Hydroxygruppen mit einer Gruppe

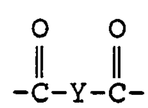

substituiert ist, in der Y wie vorstehend definiert ist, und daß mindestens einer der Reste $R^{x2}$, $R^{x3}$, $R^{x4}$ und $R^{x5}$ von Wasserstoffatom verschieden ist (sind).

4. Verbindung nach Anspruch 3, in der $R^{x1}$ eine Hydroxy- oder Benzoyloxygruppe oder einen $C_1$-$C_6$-Alkanoyloxyrest bedeutet, $R^{x2}$ ein Wasserstoffatom ist, einer der Reste $R^{x3}$ und $R^{x5}$ ein Wasserstoffatom und der andere der Reste $R^{x3}$ und $R^{x5}$ eine Hydroxy- oder Benzoyloxygruppe oder einen $C_1$-$C_6$-Alkanoyloxyrest darstellt und $R^{x4}$ eine Cyanogruppe oder ein Halogenatom bedeutet, mit der Maßgabe, daß mindestens einer der Reste $R^{x1}$, $R^{x3}$ und $R^{x5}$ eine Hydroxygruppe darstellt und daß das Wasserstoffatom einer der durch die Reste $R^{x1}$, $R^{x3}$ oder $R^{x5}$ wiedergegebenen Hydroxygruppen mit einer Gruppe

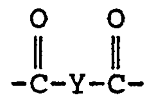

substituiert ist, in der Y wie vorstehend definiert ist.

5. Verbindung nach Anspruch 1, nämlich 3-[3-(4-Benzoyloxy-5-chlor-2-pyridyloxycarbonyl)benzoyl]-2'-desoxy-3',5'-di-O-acetyl-5-fluoruridin, 3-[3-(4-Benzoyloxy-5-chlor-2-pyridyloxycarbonyl)benzoyl]-2'-desoxy-3',5'-di-O-benzyl-5-fluoruridin, 3-[3-(2-Acetoxy-5-chlor-4-pyridyloxycarbonyl)benzoyl]-5'-desoxy-2',3'-di-O-acetyl-5-fluoruridin oder 3-[3-(6-Benzoyloxy-3-cyano-2-pyridyloxycarbonyl)benzoyl]-5'-desoxy-2',3'-di-O-acetyl-5-fluoruridin.

6. Verfahren zur Herstellung einer Verbindung, dargestellt durch die Formel

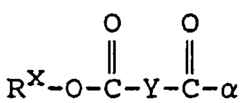

(1b)

in der $R^x$, Y und $\alpha$ wie in Anspruch 1 definiert sind, dadurch gekennzeichnet, daß

a) das Verfahren die Umsetzung einer Verbindung der Formel

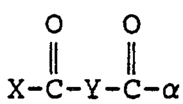

(34),

in der X ein Halogenatom bedeutet und Y und $\alpha$ wie vorstehend definiert sind, mit einer Verbindung der Formel

$R^x$-O-$R^8$ (26)

umfaßt, in der $R^x$ wie vorstehend definiert ist und $R^8$ ein Wasserstoffatom oder einen Tri-(niederalkyl)silylrest bedeutet oder

b) das Verfahren die Umsetzung einer Verbindung der Formel

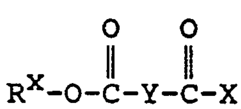

(22),

in der $R^x$ und Y wie vorstehend definiert sind und X ein Halogenatom bedeutet, mit einer Verbindung der Formel

$\alpha$H (36)

umfaßt, in der $\alpha$ wie vorstehend definiert ist.

7. Verbindung gemäß Formel 1b nach Anspruch 1 zur Verwendung bei der Behandlung von Krebs.

8. Arzneimittel, umfassend eine Verbindung gemäß Formel 1b nach Anspruch 1 und einen pharmazeutisch verträglichen Träger.